# EUROPEAN PATENT SPECIFICATION

(11) **EP 0 831 941 B1**
(45) Date of publication and mention of the grant of the patent: **04.04.2012**
(21) Application number: 96919196.4
(22) Date of filing: 06.06.1996
(51) Int. Cl.: A61K 51/04, C07D 451/02, C07D 401/00

(54) **IODINATED NEUROPROBES FOR MAPPING MONOAMINE REUPTAKE SITES**
IODIERTE NEUROSONDEN ZUM KARTIEREN VON MONOAMIN-REABSORPTIONSSTELLEN
NEUROSONDES IODEES POUR CARTOGRAPHIER LES SITES DE REABSORPTION DE LA MONOAMINE

(30) Priority: 06.06.1995 US 468575; 11.01.1996 US 584617
(43) Date of publication of application: 01.04.1998
(73) Proprietor: GE Healthcare Limited, Little Chalfont Buckinghamshire HP7 9NA (GB)
(72) Inventor: NEUMEYER, John L., Wayland, MA 01778 (US); MILIUS, Richard A., Boston, MA 02131 (US); INNIS, Robert B., Hamden, CT 06518 (US); TAMAGNAN, Gilles, Framingham, MA 01701 (US); WANG, Shaoyin, Lexington, MA 02173 (US)
(74) Representative: Canning, Lewis R.
(86) International application number: PCT/US1996/009447
(87) International publication number: WO 1996/039198

(56) References cited:
- WO-A-92/02260
- WO-A-95/01184
- WO-A-95/11901
- US-A- 5 128 118
- US-A- 5 310 912
- US-A- 5 369 113
- US-A- 5 439 666
- US-A- 5 506 359
- US-A- 5 698 179
- US-A- 5 750 089
- GOODMAN M.M. ET AL: "Synthesis of N-3-(18F)fluoropropyl-2beta-carbomethoxy-3 beta-(4- chlorophenyl)tropane: A high affinity neuroligand to map dopamine reuptake sites by PET" JOURNAL OF LABELLED COMPOUNDS AND RADIOPHARMACEUTICALS, 1994, VOL. 35, PAGE(S) 488-490, XP001053261
- BALDWIN R M ET AL: "Regional Brain Uptake and Pharmacokinetics of [I]N-omega-Fluoroalkyl-2beta-carboxy-3beta -(4-iodophenyl)nortropane Esters in Baboons" NUCLEAR MEDICINE AND BIOLOGY, ELSEVIER SCIENCE PUBLISHERS, NEW YORK, NY, US, vol. 22, no. 2, 1 February 1995 (1995-02-01), pages 211-219, XP004051853 ISSN: 0969-8051
- KUIKKA, JYRKI T. ET AL: "Iodine-123 labeled N-(2-fluoroethyl)-2.beta.-carbomethoxy-3.b eta.-(4- iodophenyl)nortropane for dopamine transporter imaging in the living human brain" EUR. J. NUCL. MED., 1995, VOL. 22, NO. 7, PAGE(S) 682-6, XP001053059
- NEUMEYER, JOHN L. ET AL: "N-.omega.-Fluoroalkyl Analogs of (1R)-2.beta.-Carbomethoxy-3.beta.-(4- iodophenyl)tropane (.beta.-CIT): Radiotracers for Positron Emission Tomography and Single Photon Emission Computed Tomography Imaging of Dopamine Transporters" J. MED. CHEM., 1994, VOL. 37, NO. 11, PAGE(S) 1558-61, XP001053024
- CANTINEAU, ROBERT ET AL: "Synthesis and biodistribution of [5-131 I]iodotropapride: A potential D2 dopamine receptor imaging agent" NUCL. MED. BIOL., 1994, VOL. 21, NO. 2, PAGE(S) 255-62, XP008000097
- LOC'H, CHRISTIAN ET AL: "Synthesis of 2.beta.-carbomethoxy-3.beta.-(4-[76Br]brom ophenyl)tropane ([76Br].beta.-CBT), a PET tracer for in vivo imaging of the dopamine uptake sites" J. LABELLED COMPD. RADIOPHARM., 1995, VOL. 36, NO. 4, PAGE(S) 385-92, XP001053077
- SCHEFFEL, U. ET AL: "Dopamine transporter imaging with novel, selective cocaine analogs" NEUROREPORT, 1992, VOL. 3, NO. 11, PAGE(S) 969-72, XP001053057
- ABI-DARGHAM ANISSA ET AL: "SPECT imaging of dopamine transporters in human brain with iodine-123-fluoroalkyl analogs of beta-CIT." JOURNAL OF NUCLEAR MEDICINE, vol. 37, no. 7, 1996, pages 1129-1133, XP001052999 ISSN: 0161-5505
- MOZLEY P D ET AL: "IPT SPECT imaging in healthy volunteers: Evaluating changes in the dopamine reuptake transporter with normal aging." JOURNAL OF NUCLEAR MEDICINE, vol. 36, no. 5 SUPPL., May 1995 (1995-05), page 32P, abstract No. 123 XP001052997 & 42nd Annual Meeting of the Society of Nuclear Medicine;Minneapolis, Minnesota, USA; June 12-15, 1995 ISSN: 0161-5505
- MOZLEY P D ET AL: "The dosimetry of (I-123)IPT: A cocaine analog for imaging the dopamine reuptake transporter." JOURNAL OF NUCLEAR MEDICINE, vol. 36, no. 5 SUPPL., May 1995 (1995-05), page 183P, abstract No. 826, XP001052998 42nd Annual Meeting of the Society of Nuclear Medicine;Minneapolis, Minnesota, USA; June 12-15, 1995 ISSN: 0161-5505
- GOODMAN MARK M ET AL: "Synthesis and characterization of radioiodinated N-(3-iodopropen-1-yl)-2-beta-carbomethoxy- 3-beta-(4-chlorophenyl)trop anes: Potential dopamine reuptake site imaging agents." JOURNAL OF MEDICINAL CHEMISTRY, vol. 37, no. 10, 1994, pages 1535-1542, XP002023291 ISSN: 0022-2623
- GOODMAN M M ET AL: "Synthesis, pharmacologic characterization, biological evaluation and primate imaging of fluorine-18 labeled 2-beta-(R,S) carbo-(2'-fluoroisopropoxy)-3-beta-(4-chlo rophenyl)-tropane (FIPCT): A selective PET radioligand for mapping dopamine reuptake sites." JOURNAL OF LABELLED COMPOUNDS AND RADIOPHARMACEUTICALS, vol. 37, December 1995 (1995-12), pages 58-60, XP001053054 & Eleventh International Symposium on Radiopharmaceutical Chemistry;Vancouver, British Columbia, Canada; August 13-17, 1995 ISSN: 0362-4803
- SWAHN C -G ET AL: "11C- and 123I-labelled 2-beta-carbomethoxy-3-beta-(4-iodophenyl)- tro pane (beta-CIT) derivatives and their labelled metabolites in monkey and human plasma determined by gradient HPLC." JOURNAL OF LABELLED COMPOUNDS AND RADIOPHARMACEUTICALS, vol. 37, December 1995 (1995-12), pages 706-708, XP001053050 & Eleventh International Symposium on Radiopharmaceutical Chemistry;Vancouver, British Columbia, Canada; August 13-17, 1995 ISSN: 0362-4803
- LUNDKVIST C ET AL: "Synthesis of 11C- or 18F-labelled analogues of beta-CIT, labelling in different positions and PET evaluation in cynomolgus monkeys." JOURNAL OF LABELLED COMPOUNDS AND RADIOPHARMACEUTICALS, vol. 37, December 1995 (1995-12), pages 52-54, XP001053029 & Eleventh International Symposium on Radiopharmaceutical Chemistry;Vancouver, British Columbia, Canada; August 13-17, 1995 ISSN: 0362-4803
- BOJA J W ET AL: "HIGH POTENCY COCAINE ANALOGS NEUROCHEMICAL IMAGING AND BEHAVIORAL STUDIES" ANNALS OF THE NEW YORK ACADEMY OF SCIENCES, 1992, pages 282-291, XP001053031 ISSN: 0077-8923
- AL-TIKRITI M S ET AL: "SPECT imaging and ex vivo autoradiographic evaluation of (123I)isopropyl beta-CIT of monoamine uptake sites in baboon." SOCIETY FOR NEUROSCIENCE ABSTRACTS, vol. 19, no. 1-3, 1993, page 936, abstract no. 385.11, XP001053039 & 23rd Annual Meeting of the Society for Neuroscience;Washington, D.C., USA; November 7-12, 1993 ISSN: 0190-5295
- KOZIKOWSKI ALAN P ET AL: "Structure-activity relationship studies of N-sulfonyl analogs of cocaine: Role of ionic interaction in cocaine binding." JOURNAL OF MEDICINAL CHEMISTRY, vol. 37, no. 20, 1994, pages 3440-3442, XP002197763 ISSN: 0022-2623

## Description

### FIELD OF THE INVENTION

This invention relates to neuroprobes for mapping monoamine reuptake sites in the brain, and particularly to neuroprobes that can also serve as radiotracers for use in single-photon emission computed tomography (SPECT) and positron emission tomography (PET) for imaging of such reuptake sites.

### BACKGROUND OF THE INVENTION

A brain consists of a plurality of neurons that interact by exchanging chemical messengers. Each neuron generates neuro-chemicals, referred to as neurotransmitters; neurotransmitters act at sites on the cellular membrane of a neuron, the sites being referred to as receptors. Receptors are associated with either ion channels through the cellular membrane or secondary neurochemical messenger systems. By contrast, reuptake sites are molecular complexes which transport chemicals across the cellular membrane of a neuron. When a neurotransmitter has served its function, it is removed from the vicinity of the receptor by being bound to a reuptake site which transports the neurotransmitter to the interior of the neuron.

Just as there are many specialized neurons in the brain, there are also a variety of neurotransmitters, associated receptors, and reuptake sites. The distribution of specialized neurons depends upon the particular organism under study, and the state of health of that organism.

A neuron can be classified according to the type of neurotransmitter that it uses to communicate with other neurons. Certain types of neurons can be found predominantly in particular regions of the brain. For example, the striatal region of a mammalian brain is innervated by neurons using dopamine as a neurotransmitter. The striatum also contains a large number of non-dopaminergic neurons that have dopamine receptors. Certain compounds, such as cocaine, have a preferential affinity for dopamine reuptake sites, and therefore tend to bind to such reuptake sites. The effect of a molecule such as cocaine upon a dopamine reuptake site is to inhibit reuptake of the neurotransmitter dopamine, leaving more dopamine available in the vicinity of the dopamine receptors.

In certain neurological diseases, such as Parkinson's disease, distinct groups of neurons lose their normal physiological functioning. Consequently, the abnormal neurons may behave differently in the presence of some neurotransmitters, and may also produce neurotransmitters in a manner that differs from a healthy neuron.

The major neurotransmitters, dopamine, norepinephrine, and serotonin, are referred to collectively as the monoamine neurotransmitters. Many neurons have receptors adapted to receive at least one of these neurotransmitters. Parkinson's disease is caused by the degeneration of some of the dopaminergic neurons in the brain. The neurons lost in Parkinson's disease have a large number of dopamine reuptake sites; cocaine and chemical analogs of cocaine have an affinity for such reuptake sites.

A radioisotope is commonly incorporated in molecules that have a demonstrated binding affinity for a particular type of neuro-receptor, and such molecules are commonly used as neuroprobes. The localization of neuroprobes can be used to find specialized neurons within particular regions of the brain. It is also known that a neurological disease can be detected by observing abnormal binding distributions of a neuroprobe. Such abnormal binding distributions can be observed by incorporating a radionuclide within each molecule of the neuroprobe with a high binding affinity for the particular reuptake sites of interest. Then, an imaging technique can be used to obtain a representation of the in vivo spatial distribution of the reuptake sites of interest.

In single photon emission computed tomography (SPECT) imaging, the most commonly used radionuclides are heavy metals, such as ^{99m}Tc. Heavy metals are very difficult to incorporate into the molecular structure of neuroprobes because such probes are relatively small molecules (molecular weight less than 400).

In positron emission tomography (PET), the radiohalide ¹⁸F (fluorine) is commonly used as a substitute for H (hydrogen) in radiopharmaceuticals because it is similar in size. Not all halogens will work, however. For example, I (iodine) is much larger than both H and F, being approximately half the size of a benzene ring. However, due to the small size of typical radiopharmaceuticals for use as neuroprobes, the presence of iodine markedly changes the size of the compound, thereby altering or destroying its biological activity.

In addition, the presence of iodine in a neuroprobe tends to increase its lipophilicity, and therefore increases the tendency of the neuroprobe to engage in non-specific binding. For example, paroxetine is a drug with high affinity and selectivity for serotonin reuptake sites, and [³H]paroxetine has been shown in rodents to be a useful in vivo label (Scheffel, U. and Hartig, PR. J. Neurochem., 52: 1605-1612, 1989). However, several iodinated analogs of this compound with iodine attached at several different positions had unacceptably low affinity, in fact being one tenth of the affinity of the parent compound. Furthermore, when the iodinated compound was used as an in vivo radiolabeled neuroprobe, non-specific binding activity was found to be so high that no measurable portion of the brain uptake appeared to be specifically bound to the serotonin reuptake site. Thus, the iodinated form of paroxetine is not useful as an in vivo probe.

The addition of iodine to a neuroprobe can unfavorably alter its biological properties. For example, tomoxetine has high affinity and selectivity for norepinephrine reuptake sites. However, when tomoxetine is iodinated, e.g. to form R-4-iodoto-moxetine, the resulting labeled compound has low affinity for such reuptake sites, and relatively high affinity for serotonin reuptake sites. In vivo labeling studies have shown that it is an unacceptably poor probe even for the serotonin reuptake sites because it exhibits low total brain uptake and immeasurably low specific uptake.

An iodinated compound can be useful as an in vitro probe, but may be useless as an in vivo probe, because an in vivo probe must meet the requirements associated with intravenous administration of the probe to a living subject. Reasons for the loss of in vivo utility include the fact that the compound may be metabolized too quickly, that it may not cross the blood-brain-barrier, and that it may have high non-specific uptake into the lipid stores of the brain. In vitro homogenate binding studies remove these obstacles by isolating the brain tissue from hepatic metabolic enzymes, by homogenizing the brain tissue so as to destroy the blood-brain-barrier, and by diluting the brain tissue so as to decrease the concentration of lipids in the assay tube. Accordingly, it cannot be assumed that a probe will be useful in both in vivo and in vitro modalities.

An in vivo SPECT probe was developed by iodinating cocaine. However, this probe shows a binding affinity and specificity no better than cocaine itself, which is inadequate for purposes of SPECT imaging.

US 5,310,912 , US 5,439,666 and WO 95/01184 disclose radioiodinated neuroprobes for mapping monoamine reuptake sites which comprise tropanes with N-functionalised monofluoroalkyl substituents. Kuikka et al [Eur.J.Med.Chem., 22(7), 682-686 (1995)] disclose similar compounds, where the tropane has an N-fluoropropyl or N-fluoroethyl substituent. Goodman et al [J.Lab.Comp.Radiopharm., 35, 488-490 (1994)] discloses the synthesis of *N*-3-β-[¹⁸F]fluoropropyl-2β-carbomethoxy-3β-(4-chlorophenyl)tropane, a PET imaging agent for dopamine reuptake sites. WO 92/02260 discloses radioiodinated tropane-carboxylic acid derivatives for use in imaging dopamine and serotonin transporters in the brain *in vivo.* US 5,698,179 discloses neuroprobes for mapping monoamine reuptake sites, based on tropanes, where the tropane has an N-(CH₂)ₙOSO₂R³ and R³ is alkyl, substituted alkyl, alkenyl, substituted alkenyl, aryl, substituted aryl, and heterocyclic; and n=1-6.

### SUMMARY OF THE INVENTION.

The invention is directed to a kit for preparing an ¹⁸F-radiolabelled neuroprobe for mapping monoamine reuptake sites, the kit comprising a non-radioactive precursor of the formula: 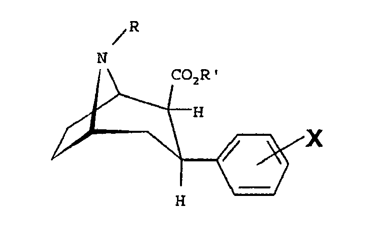 wherein R = (CH₂)ₙOSO₂R³;
wherein R³ = alkyl, substituted alkyl, alkenyl, substituted alkenyl, aryl,
substituted aryl, and heterocyclic; and n = 1- 6;
R' = C_{w}H_{2w+1} wherein w = 0-6; and
X = Cl, Br, F, I or an aryl group;
wherein the precursor is reacted in the presence of ¹⁸F and wherein the ¹⁸F is selected from the group consisting of Na¹⁸F, K¹⁸F, and Cs¹⁸F.

Both the radiostable and radioactive variants of the neuroprobe of the invention are useful for human and non-human research. For example, in vivo and in vitro experiments can be performed using the compounds of the invention to study monoamine reuptake sites generally, and cocaine binding sites in particular.

### DETAILED DESCRIPTION OF THE INVENTION

Metabolically stable cocaine analogs such as 2β-carbomethoxy-3β-(4-iodophenyl)-tropane, an iodine-containing analog of β-CIT (also designated RTI-55), have high affinities for dopamine and serotonin reuptake sites in brain.

[¹²³I]-β-CIT appears to be a useful SPECT tracer of the dopamine and serotonin reuptake sites. Brain uptake and washout are relatively slow in comparison to cocaine itself and are consistent with the metabolically resistant chemical structure of β-CIT and the location of the radioiodine in a chemically stable position. Striatal uptake appears to largely represent labeling of the dopamine reuptake site, whereas that in the midbrain is largely associated with the serotonin reuptake site. The high ratios of striatal to cerebellar activity of [¹²³I]-β-CIT are consistent with low non-specific uptake of the tracer, and suggest that [¹²³I]-β-CIT may be a useful clinical marker of dopaminergic deficiencies in Parkinson's disease.

. N-Demethylation of compound 6 was accom-plished by conversion to its 2,2,2,-trichloroethyl carbamate followed by reduction (Zn/acetic acid) to yield compound 8 by the procedure previously described by Milius, R.A., et al., J. Med. Chem. 34 1728-1731 (1991), followed by iodination to yield nor-CIT (compound 4), which was isolated as a yellow crystalline solid (free base 48% from com-pound 6): mp 149-151°C; [α]²⁵D -67.4° (c = 1, CHCl₃).

### EXAMPLES OF SYNTHESES

### Example 1. 2-β-Carbomethoxy-3-β-(4-iodophenyl)tropane

A mixture of 2-β-carbomethoxy-3-β-phenyltropane (See Example 1A below and Milius et al. J. Med. Chem., 1991, 34, 1728) (2.9 g, 11.5 mmol) and I₂ (3 g, 11.8 mmol) in 25 ml of glacial acetic acid was stirred and treated dropwise with a mixture of 4.7 mL of concentrated nitric acid and 4.7 mL of concentrated sulfuric acid. The reaction mixture was heated to 55°C and stirred for 2 hours, then cooled to room temperature and poured onto ice (100 g) and filtered. The pH of the filtrate was adjusted to 9.5 by the addition of concentrated ammonium hydroxide at 0-5°C. The resulting precipitate was removed by filtration and dissolved in methylene chloride (250 ml). The filtrate was extracted with two 50 ml portions of methylene chloride. The extracts and solution of precipitate were combined, washed with brine (50 ml) and dried over magnesium sulfate. After the removal of the solvent, 3.9 g (90.4%) of 2-β-carbomethoxy-3-β-4-iodophenyltropane free base was obtained as an oil.

The free base was dissolved in methanol (20 ml) and combined with 1.5 g of D-(-)tartaric acid in 20 ml of methanol. After the removal of methanol under reduced pressure, the residue was recrystallized from methanol ether (3:1) to give 2-β-carbomethoxy-3-β-(4-iodophenyl)tropane D-tartrate salt as white crystals, m.p. 72-74°C. C₁₆H₂₀NO₂I.C₄H₆O₆. Calculated: C: 44.88, H: 4.89, N: 2.62. Found: C: 44.70, H: 4.94, N: 2.57. [alpha]_{D}²² = -87.7°(c=0.3, CH₃OH).

### Example 1A. 2-β-Carbomethoxy-3-β-phenyltropane

A 2M ethereal solution of phenylmagnesium bromide (83 mL, 166 mmol) in a 500-mL 3-neck round-bottom flask equipped with mechanical stirrer, addition funnel, and nitrogen inlet tube was diluted with 83 mL of anhydrous diethyl ether and cooled to -20°C under an atmosphere of dry nitrogen. A solution of anhydroecgonine methyl ester, prepared from cocaine (1) (15 g, 82.8 mmol) in anhydrous ether (75 mL) was added dropwise. The heterogeneous mixture was stirred for 1 h at -20°C, then poured into an equal volume of ice and water, and acidified by the dropwise addition of 2 M HCl. The aqueous layer was made basic by the addition of concentrated ammonium hydroxide, saturated with NaCl, and extracted with diethyl ether. The combined extracts were dried (Na₂SO₄) and concentrated in vacuo to give a brown oil. Bulb to bulb distillation (70°C, 0.9 Torr) of the crude product gave a pale yellow oil (16 g, 70%). TLC analysis of the oil (silica, pentane/diethyl ether/2-propylamine, 15:5:0.8) showed it to be a mixture of the C-2 alpha and beta epimers. The beta isomer was isolated by silica gel chromatography (pentane: diethyl ether: isopropyl amine, 70:30:3). m.p. 63-66°C (lit: 62-64, 5°C: Clarke et al. J. Med. Chem.. 16: 1260 (1973)).

### Example 2. 2-α-Carbomethoxy-3-β-iodophenyltropane

The mixture of α- and β-2-carbomethoxy-3-β-iodophenyltropanes prepared as described in Example 1 were separated by silica gel chromatography as described in Example 1. Fractions containing the α-2-carbomethoxy-3-β-iodophenyl-tropane were pooled and concentrated in vacuo. The free base thus obtained was treated with naphthalene-1,5-disulfonic acid. The crude salt was recrystallized from acetonitrile to give the 2-α-carbomethoxy-3-β-iodophenyltropane naphthalene-1,5-di-sulfonate salt, m.p. 166-168°C. C₁₆H₂₀NO₂, · C₁₀H₆(SO₃H)₂· 2H₂O. Calculated: C:40.01, H:4:55, N:1.97, I:17.90; Found: C:43.94, H:4.55, N:1.91, I:17.99.

### Example 3. 2-β-Carbomethoxy-3-β-(4-iodophenyl)nortropane.

A solution of 2-β-carbomethoxy-3-β-(4-iodophenyl)tropane (410 mg, 1.5 mmol) in toluene (20 mL) was treated with of 2,2,2-trichloroethyl chloroformate (1 mL, 7.3 mmol). The mixture was heated at 120°C for 1 hour, cooled to room temperature, and evaporated to dryness in vacuo. The residue was partitioned between methylene chloride and water. The organic layer was separated, dried (Na₂SO₄), and concentrated in vacuo to give the trichloroethyl chloroformate as a dry foam. The crude carbamate was dissolved in 50% aqueous acetic acid, treated with 200 mg (0.0067 g-atom) of zinc dust, and stirred at room temperature for 16 hours. The reaction mixture was filtered adjusted to pH 7 with concentrated ammonium hydroxide, saturated with NaCl, and extracted with diethyl ether. The extracts were combined, dried (Na₂SO₄), and concentrated in vacuo. The residue was purified by flash chroma-tography (silica, pentane/diethyl ether/isopropylamine, 3:7:0.7) to afford 2-β-carbomethoxy-3-β-(4-iodophenyl)nortropane, which was isolated as a yellow crystalline solid, m.p. 149-151°C; [alpha]²⁵_{D} -67.4° (c = 1, CHCl₃).

### Example 4

To avoid the necessity of storing a radioactive neuroprobe, it is useful to provide a kit containing the non-radioactive precursor compound.

Then, the non-radioactive precursor compound can be oxidized in the presence of a suitable radioactive compound, such as
a reagent containing ¹⁸F of the formula FCₙH₂ₙX, where n=0-6 and X is a leaving group, to prepare the neuroprobe at its time and place of use. labeled neuroprobes can be used in PET imaging.

Both the radiostable and radioactive variants of the neuroprobe of the invention are useful for human and non-human research. For example, in vivo and in vitro experiments can be performed using the compounds of the invention to study the dopamine transporter generally, and cocaine binding sites in particular.

The functional substituents of N-8 include leaving groups, such as sulfonate esters. Sulfonate esters, such as mesylates, tosylates and triflates (trifluoromethanesulfonates) are particularly useful leaving groups.

### Example 5

### General procedure for N-Alkylation of Nor-β-CIT

N-Alkylation reactions are typically carried out with 0.27 mmol of nor-β-CIT (compound 4). To a solution of nor-β-CIT and tri-ethylamine (46 mmol) in absolute EtOH or anhydrous toluene is added the appropriate alkyl bromide (0.4 mmol) and KI (10 mg). The mixture is refluxed under nitrogen from 1 to 24 hours depending on alkyl bromide monitoring by thin layer chromatography (TLC) to the completion of reaction. The solvent is then removed under reduced pressure and the residue is passed through a silica gel column (eluted with hexane/ether/TEA) to yield the pure compounds. Examples 10-14 describe alkylation reactions of the N-8 group.

### Example 6. Synthesis of N-(2-Hydroxyethyl)-β-CIT

Nor-β-CIT (5 mmol) is dissolved in ethanol (30 mL), together with 2-bromoethyltetrahydropyran (7.5 mmol), triethylamine (0.76 g) and potassium iodide (250 mg). The mixture is heated at reflux under nitrogen for 16h. When the reaction is completed, the solvent is removed at reduced pressure and the residue is passed through a silica gel column eluting with hexane/ether/triethylamine (15/80/5). The fractions containing product are collected and concentrated to give the pure protected compound. This compound is stirred with H₂O (10 mL), THF (10 ml) and acetic acid (30 mL) during 20 h at 60°C. The solvent is removed, and the residue is basified with NH₄OH and extracted with dichoromethane. The organic layer is dried over MgSO₄ and concentrated. The residue is passed through a silica gel column eluting with hexane/ether/triethylamine (10/80/10). The fractions containing product are collected and concentrated to give 1.3 g of product as a colorless oil.

¹H NMR (300 MHz, CDCl₃): δ 7.58 (d, J=8.4Hz, 2H) ; 6.99 (d, J=8.4Hz, 2H); 3.50 (m, 4H); 3.49 (s, 3H); 2.94 (m, 1H); 2.88 (m, 1H); 2.63 (m, 2H); 2.42 (m, 2H); 2.05 (m, 2H); [α]_{D}²⁰ -34.06° (c, 0.3, MeOH).

### Example 7. Synthesis of N-[3-(p-Tolylsulfonyloxyropyl)]-2β-carbomethoxy-3β-(4'-iodopheny)nortropane

A solution of N-(3-hydroxypropyl)nor-β-CIT (150 mg, 0.35 mmol), pyridine (100 mg), and p-toluenesulfonyl chloride (100 mg) in chloroform (15 ml) is stirred at room temperature for 4 hr, diluted with water (50 ml), and extracted with chloroform (100 ml). The organic layer is concentrated under reduced pressure. The residue is purified by flash chromatography on silica gel, eluting with hexane/ether/TEA (10/70/0.1) to give 51 mg of product as an oil. The yield is approximately 25%.

¹H NMR (CDCl₃): δ 1.62-1.80 (m, 3H), 2.01-2.18 (m, 3H), 2.45 (s, 3H), 2.62 (m, 1H), 2.91 (m, 1H), 3.43 (m, 1H), 3.51 (s, 3H), 3.80 (m, 1H), 4.36-4.52 (m, 2H), 6.99-7.58 (ABq, 4H), and 7.55-7.80 (ABq, 4H). Elemental analysis calculated for C₂₅H₃₀NO₅IS·1/2H₂O: C, 50.68; H, 5.27; N, 2.36; Found: C, 50.64; H, 5.45; N, 2.10.

### Example 8. Synthesis of N-(3-hydroxypropyl)-2β-carbomethoxy-3β-(4'-iodopheny)tropane

A solution of nor-β-CIT (250 mg, 0.67 mmol), 3-bromopropanol (300 mg, 2.13 mmol) and triethylamine (0.5 ml) in toluene (20 ml) is refluxed under a dry nitrogen atmosphere for 4 hr, cooled and filtered. The separated residue is washed with toluene (2x2 ml). The combined filtrate and washings are concentrated under reduced pressure. The residue was purified by flash chromatography on silica gel, eluting with hexane/ether/TEA (10/7/0.1) to give 168 mg of product as a liquid. Yield is approximately 58%.

¹H NMR (CDCl₃): δ 1.62-1.80 (m, 5H), 1.98-2.18 (m, 2H), 2.36-2.42 (m, 2H), 2.51-2.63 (m), 2.90-3.02 (m, 2H), 3.40 [s(br), m, 1H], 3.70 [s(br), 1H], 4.44-4.59 (m, 2H), 7.00-7.03 and 7.57-7.60 (ABq, 4H). Elemental analysis calculated for C₁₈H₂₄NO₃F: C, 50.36; H, 5.64; N, 3.26; Found: C, 50.35; H, 5.57; N, 3.19.

### Example 9. Synthesis of N-[3-(methanesulfonyloxy)propyl]-2β-car-bomethoxy-3β-(4'-iodopheny)nortropane

To a solution of N-(3-hydroxypropyl)nor-β-CIT (380 mg, 0.88 mmol) and 2,6-lutidine (150 µl) in chloroform (25 ml) is added methane-sulfonyl chloride (152 mg, 1.33 mmol) at 0°C. The solution is stirred at 0°C for 2 hr and then a second portion of methane-sulfonyl chloride is added and stirring is continued at room temperature for an additional 4 hr. After removal of the solvent, the residue is purified by flash chromatography on silica gel, eluting with hexane/ether/TEA (10/7/0.1) to give 190 mg of product as an oil. Yield is approximately 40%.

¹H NMR (CDCl₃) : δ 1. 62-180 (m, 3H), 2.01-2.18 (m, 3H), 2.45 (s, 3H), 2.62 (m, 1H), 2.91 (m, 1H), 3.04 (s, 3H), 3.43 (m, 1H), 3.51 (s, 3H), 3.80 (m, 1H), 4.36-4.31 (m, 2H) and 6.99-7.58 (ABq, 4H).

Elemental analysis calculated for C₁₉H₂₆NO₅IS 1/2H₂O: C, 43.43; H, 5.37; N, 2.67; Found: C, 43.12; H, 5.15; N, 2.58.

### Example 10 Preparation of Radiolabeled Compounds from the Nonradioactive Intermediates

In general, the leaving group attached to the moiety at N-8 is capable of being displaced by a radionuclide such as ¹⁸F. The chemistry of the reaction is based on nucleophilic substitution of [¹⁸F]fluoride into an activated precursor dissolved in a solvent.

In general, the precursor is an N-substituted β-CIT derivative that includes a leaving group on the moiety attached to N-8. The leaving group is preferably a mesylate, or another sulfonate ester, such as tosylate or triflate.

Solvents used in the reaction are preferably anhydrous, polar, aprotic solvents, such as acetonitrile, dimethylsulfoxide, dimethylforma-mide, N-methylpyrrolidone, hexamethylphosphoric triamide, and the like.

The radioisotope is generated in minute quantities and generally requires an auxiliary reagent to dissolve in the solvent and participate in the chemical reaction. The solubilizing agent can be any agent capable of solubilizing radionuclides that takes the form M⁺X⁻. M⁺ is preferably the complex of potassium and 4,7,13,16,21,24-hexaoxa-1,10-diazabicyclo[8.8.8]hexacosane or an alkali metal ion.

### Example 11. Preparation of [¹⁸F]-8-(3-fluoropropyl)-2β-CIT from N-(3-mesyloxypropyl)-N-nor-β-CIT

An aqueous solution of [¹⁸F]fluoride ions (0.5 mL) is mixed with 4,7,13,16,21,24-hexaoxa-1,10-diazabicyclo[8.8.8]hexacosane (10 mg) and potassium carbonate (1 mg) in a borosilicate glass vessel of 5 mL capacity. The vessel is partially immersed in an oil bath thermostated to 100°C, and the solution evaporated to dryness using a stream of nitrogen. An aliquot of anhydrous acetonitrile (1 mL) is added to the reaction vessel and allowed to evaporate under the nitrogen flow. This addition/evaporation step is performed a second time. Heating of the vessel is continued for approximately one minute after evaporation of the second aliquot.

The vessel is next raised above the oil bath. To the residue is added a solution of N-(3-mesyloxypropyl)-N-nor-β-CIT (2 mg; see Example 21) in anhydrous acetonitrile (1 mL). The vessel is reim-mersed in the oil bath so that the solvent achieves gentle reflux. Heating is continued for approximately five minutes, and then the vessel is cooled to room temperature.

The reaction mixture is concentrated to near dryness under a stream of nitrogen. The residue is dissolved in 3:1 methanol-water (0.5 mL) and injected into a high pressure liquid chromatograph fitted with a 10 x 250 mm column packed with octadecyl-functional-ized silica and eluted with 3:1 methanol-water at 4 mL/min. The effluent is collected in test tubes at 0.5 minute intervals. The fractions containing N-(3-[¹⁸F] fluoropropyl) -N-nor-β-CIT are com-bined, evaporated to dryness, and redissolved in USP sodium chlor-ide injection containing 5% by volume USP ethanol and 0.1 mM L-ascorbic acid.

## Claims

1. A kit for preparing a **an ¹⁸F**-radiolabelled neuroprobe for mapping monoamine reuptake sites, the kit comprising:
a **non-radioactive** precursor of the formula: 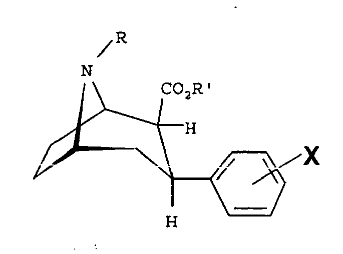 wherein R = (CH₂)ₙOSO₂R³;
wherein R³ = alkyl, substituted alkyl, alkenyl, substituted alkenyl, aryl, substituted aryl, and heterocyclic; and n = 1- 6;
R' = C_{w}H_{2w+1} wherein w = 0-6 ; and
X= or an aryl group;
**wherein the precursor is reacted in the presence of ¹⁸F and wherein the ¹⁸F is selected from the group consisting of Na ¹⁸F, K¹⁸F, and Cs¹⁸F.**

2. The kit of claim 1, wherein said ¹⁸F is complexed with a reagent of the formula M⁺X⁻, wherein:
M⁺ = a complex of 4,7,13,16,21,24-hexaoxa-1,10-diazabicyclo[8.8.8]hexacosane and one of K, Na, Ce, Ru, a tetraalkylammonium ion, and an ion exchange resin functionalized with quaternary amine groups; and
X⁻ = carbonate, bicarbonate, hydroxide, and formate.

## Patentansprüche

1. Kit zur Herstellung einer mit ¹⁸F radioaktiv markierten Neurosonde zum Kartieren von Monoaminwiederaufnahmestellen, wobei das Kit umfasst:
einen nichtradioaktiven Präkursor der Formel: 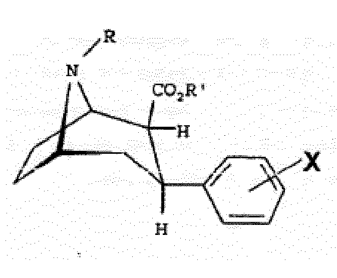 worin R = (CH₂)ₙOSO₂R³;
worin R³ = Alkyl, substituiertes Alkyl, Alkenyl, substituiertes Alkenyl, Aryl, substituiertes Aryl, und heterocyclisch; und n = 1 - 6;
R' = C_{w}H_{2w+1}, worin w = 0 - 6; und
X = Cl, Br, F, I oder eine Arylgruppe;
wobei der Präkursor bei Vorhandensein von ¹⁸F umgesetzt wird und wobei das ¹⁸F aus jener Gruppe gewählt ist, die sich aus Na¹⁸F, K¹⁸F und Cs¹⁸F zusammensetzt.

2. Kit nach Anspruch 1, wobei das ¹⁸F mit einem Reagens der Formel M⁺X⁻ komplexiert wird, worin:
M⁺ = ein Komplex aus 4,7,13,16,21,24-Hexaoxa-1,10-diazabicyclo[8.8.8]hexacosan und einem von K, Na, Ce, Ru, einem Tetraalkylammoniumion und einem Ionenaustauschharz, funktionalisiert mit quaternären Amingruppen; und
X⁻ = Carbonat, Bicarbonat, Hydroxid und Formiat.

## Revendications

1. Kit de préparation d'une neurosonde radiomarquée par ¹⁸F afin d'assurer la cartographie de sites de ré-absorption de monoamine, le kit comprenant :
un précurseur non radioactif selon la formule suivante ; 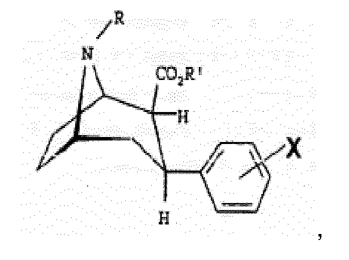
dans laquelle R = (CH₂)ₙOSO₂R³,
où R³ = un alkyle, un alkyle substitué, un alkényle, un alkényle substitué, un aryle, un aryle substitué, et hétérocyclique ; et n = 1 à 6 ;
R' = C_{w}H_{2w+1} où w = 0 à 6 ; et
X = Cl, Br, F, I ou un groupe aryle ;
dans lequel le précurseur est mis en réaction en la présence de ¹⁸F et dans lequel ¹⁸F est sélectionné à partir du groupe constitué par Na ¹⁸F, K¹⁸F, et Cs¹⁸F.

2. Kit selon la revendication 1, dans lequel ledit ¹⁸F est complexé avec un réactif de formule M⁺X⁻, dans laquelle :
M⁺ = un complexe de 4,7,13,16,21,24-hexaoxa-1,10-diazabicyclo[8.8.8]hexacosane et de l'un parmi K, Na, Ce, Ru, un ion tétra-alkylammonium, et une résine d'échange d'ion fonctionnalisé avec des groupes amines quaternaires ; et
X⁻ = un carbonate, un bicarbonate, un hydroxyde, et un formate.
